## Europäisches Patentamt
## European Patent Office
## Office européen des brevets

(19)

(11) Veröffentlichungsnummer : **0 192 640**
**B1**

(12) **EUROPÄISCHE PATENTSCHRIFT**

(45) Veröffentlichungstag der Patentschrift :
15.03.89

(21) Anmeldenummer : 84903631.4

(22) Anmeldetag : 22.09.84

(86) Internationale Anmeldenummer :
PCT/EP 84/00294

(87) Internationale Veröffentlichungsnummer :
WO/8601406 (13.03.86 Gazette 86/06)

(51) Int. Cl.⁴ : **A 61 K 31/70, A 61 K 47/00**

(54) SUCRALFAT-SUSPENSION.

(30) Priorität : 22.08.84 DE 3430809

(43) Veröffentlichungstag der Anmeldung :
03.09.86 Patentblatt 86/36

(45) Bekanntmachung des Hinweises auf die Patenterteilung : 15.03.89 Patentblatt 89/11

(84) Benannte Vertragsstaaten :
AT BE CH DE FR GB LI NL SE

(56) Entgegenhaltungen :
EP–A– 0 055 313
EP–A– 0 107 209
GB–A– 2 120 942
US–A– 3 692 898
US–A– 4 136 173

(73) Patentinhaber : MERCK PATENT GESELLSCHAFT
MIT BESCHRÄNKTER HAFTUNG
Frankfurter Strasse 250 Postfach 4119
D-6100 Darmstadt (DE)

(72) Erfinder : HANSTEIN, Ullrich, Dr.
Schleifmühlenweg 4
D-6109 Mühltal (DE)
Erfinder : BAUER, Lothar
Mühlbergstrasse 92
D-6102 Pfungstadt (DE)

Anmerkung : Innerhalb von neun Monaten nach der Bekanntmachung des Hinweises auf die Erteilung des
europäischen Patents im Europäischen Patentblatt kann jedermann beim Europäischen Patentamt gegen das erteilte
europäische Patent Einspruch einlegen. Der Einspruch ist schriftlich einzureichen und zu begründen. Er gilt erst als
eingelegt, wenn die Einspruchsgebühr entrichtet worden ist (Art. 99(1) Europäisches Patentübereinkommen).

**Beschreibung**

Die Erfindung betrifft pharmazeutische Zubereitungen in Form von Suspensionen, die Sucralfat als pharmazeutischen Wirkstoff enthalten.

Sucralfat (Ulcogant[(R)]) ist ein basisches Aluminium-Saccharose-Sulfat. Es ist aus der DE-OS 15 68 346 bekannt und wird in der Humanmedizin zur Linderung der Beschwerden bei Ulcus ventriculi et duodeni und zur Beschleunigung der Ulcusabheilung verwendet. Darüberhinaus ist eine vorteilhafte Wirkung von Sucralfat bei der Bekämpfung von Emesis und/oder Diarrhoe in der Veterinärmedizin in der Deutschen Patentanmeldung P 33 22 078 beschrieben.

Die Wirkung von Sucralfat ist charakterisiert durch pepsinbindende und antacide Effekte. Das sehr gut verträgliche Sucralfat entfaltet seine Wirkung im sauren Medium des Verdauungstraktes, insbesondere bei pH-Werten unter 4, wobei es die Schleimhäute des Magens und Zwölffingerdarmes mit einer schützenden Schicht auskleidet. Durch ein bevorzugtes Bindevermögen an angegriffene Schleimhautbereiche kommt es dort zu einem verstärkten Schutz und zu einer beschleunigten Ulcusabheilung sowie einer Regeneration der Schleimhaut und ihrer Funktionen.

Sucralfat enthaltende Zubereitungen kamen bislang üblicherweise nur in Form von festen Darreichungsformen wie Tabletten, Granulaten oder Pulvern zur Anwendung. Für die spezielle Wirkungsweise von Sucralfat, insbesondere im Hinblick auf ein schnelles und vollständiges Auskleiden der Schleimhäute im Verdauungstrakt, wären jedoch flüssige Zubereitungen, etwa in Form von Suspensionen, vorteilhaft. Es ist zwar möglich, die handelsüblichen festen Darreichungsformen vor der Anwendung beispielsweise in Wasser aufzuschlämmen und in dieser Form einzunehmen. Dies erweist sich aber als umständlich und wird häufig für das geschmackliche Empfinden des Einnehmenden als unangenehm empfunden, ist somit also wenig praktikabel. Versuche, Sucralfat enthaltende Fertigarzneimittel in Form von Suspensionen herzustellen, schlugen bislang fehl, da mit den üblichen Hilfsstoffen keine auf Dauer stabile Suspensionen zu erhalten waren. In derartigen Suspensionen war der Feststoff nach kurzer Zeit sedimentiert und derart verklumpt, daß teilweise eine Aufschüttelbarkeit nicht mehr gegeben war.

Der Erfindung lag somit die Aufgabe zugrunde, ein Verfahren zur Stabilisierung von pharmazeutischen Zubereitungen in Form von Sucralfat enthaltenden Suspensionen und eine entsprechende stabile Zubereitung als Fertigarzneimittel zu finden.

Für die Formulierung pharmazeutischer Suspensionen versucht man durch geeignete Zusätze eine Sedimentation der suspendierten Partikel zu verhindern oder zumindest so gering wie möglich zu halten. Hierdurch soll gewährleistet sein, daß das Mittel auch nach längerer Lagerung homogen und entsprechend anwendbar bleibt. Findet doch im Laufe der Zeit eine Sedimentation statt, so sollte eine gute Aufschüttelbarkeit des Festkörpers gewährleistet sein. Darüberhinaus sollten Suspensionen für die orale Anwendung ein möglichst angenehmes oder zumindest neutrales Geschmacksgefühl vermitteln. Insbesondere sollte der Feststoffanteil so eingearbeitet sein, daß dieser bzw. die Feststoffpartikel bei der Einnahme nicht als störend empfunden werden. Bei wässerigen Suspensionen können die genannten Eigenschaften üblicherweise durch Zufügen von viskositätserhöhenden Flüssigkeiten wie Glycerin, Propandiol, Sorbitlösung und/oder flüssige Polyethylenglykole, sowie durch Einarbeiten von Suspendierungs- und Verdickungsmitteln erreicht werden. Derartige die Viskosität erhöhende und eine Sedimentation der Feststoffpartikel verhindernde Mittel sind üblicherweise hochmolekulare Cellulosederivate oder Polysaccharidgummen, wie beispielsweise Carboxymethylcellulosen, Methylcellulosen, Alginate oder Traganth.

Zur Lösung der Aufgabe, eine stabile, nicht sedimentierende Sucralfat-Suspension zu entwickeln, wurden zahlreiche Versuche unternommen, die keinen oder nur geringen Erfolg brachten. Hierzu wurden die wesentlichen gängigen, in der Technologie der pharmazeutischen Suspensionen üblichen Suspendierungs- und Verdickungsmittel sowie entsprechende Hilfs- und Zusatzstoffe getestet. Es zeigte sich, daß offenbar durch die Eigenschaft des Sucralfates, im wässerigen Medium $Al^{3+}$-Ionen freizusetzen, Interaktionen mit praktisch allen verdickenden Substanzen induziert werden, die zu bisweilen drastischen Viskositätsabnahmen oder -zunahmen sowie zu Ausfällungen der Verdickungsmittel führen. Dies führt dazu, daß die Sucralfatpartikel in derartigen Suspensionszubereitungen sehr schnell unter Bildung verklumpter Niederschläge sedimentieren, sich an den Wandungen des Verpackungsmittels anhaftend niederschlagen und sich nicht mehr aufschütteln lassen.

Es wurde nun gefunden, daß sich überraschenderweise stabile Zubereitungen in Form von Sucralfat enthaltenden Suspensionen erhalten lassen, wenn man diesen Zubereitungen bezogen auf den Gehalt an Sucralfat, 1-5 Gew.% Xanthangummi und 1-12,5 Gew.% mindestens eines Peptisators zusetzt.

Gegenstand der Erfindung sind dementsprechend pharmazeutische Zubereitungen in Form von Sucralfat enthaltenden Suspensionen, die einen Gehalt, bezogen auf das Sucralfat, von 1-5 Gew.% Xanthangummi und 1-12, 5 Gew.% mindestens eines Peptisators aufweisen.

Gegenstand der Erfindung ist insbesondere eine solche pharmazeutische Zubereitung, in der der Peptisator mindestens ein Salz der Phosphor- und/oder Citronensäure ist.

Gegenstand der Erfindung ist darüberhinaus ein Verfahren zur Stabilisierung von pharmazeutischen Zubereitungen in Form von Sucralfat enthaltenden Suspensionen, wobei man diesen Zubereitungen, bezogen auf den Gehalt an Sucralfat, 1-5 Gew.% Xanthangummi und 1-12,5 Gew.% mindestens eines Peptisator zusetzt.

EP 0 192 640 B1

Xanthangummi (« Polysaccharid 1459 ») ist ein hochmolekulares Polysaccharid, das durch Fermentation von Kohlenhydraten mit Pseudomonas-Mikroben, insbesonders mit Xanthomonas campestris erhältlich ist und üblicherweise in Form seiner Alkali- und/oder Erdalkalimetallsalze vorliegt. Es ist bekannt, daß Xanthangummi unter anderem auch als Suspendierungs- und Verdickungsmittel für pharmazeutische und kosmetische Präparate verwendet werden kann (siehe H.P. Fiedler, Lexikon der Hilfsstoffe, S. 1016, 2. Aufl. (1981) oder The United States Pharmacopeia, Twentieth Revision (1980)). So ist ein basisches Aluminiumsalz von Xanthangummi als pharmazeutisches Exipiens bekannt sowie die Verwendung desselben als Suspendierungsmittel für Bariumsulfat in radiographischen Kontrastmitteln (vgl. Chemical Abstracts 79 (1973) 23582m und 81 (1973) 96461x). Andererseits sind aber auch Arbeiten bekannt, in denen Xanthangummi zur Ausflockung von Metalloxiden, -hydroxiden, -carbonaten, insbesondere auch des Aluminiums, aus Suspensionen verwendet wird (Chemical Abstracts 94 (1981) 52786w, 95 (1981) 12692b und 96 (1982) 24726z). Dem Stand der Technik konnte somit keine generelle Eignung von Xanthangummi zur Stabilisierung von Sucralfat enthaltenden pharmazeutischen Suspensionen entnommen werden. Vielmehr zeigte sich in den zahlreichen durchgeführten Versuchen, daß sich auch mit Xanthangummi, wie mit allen anderen üblichen Verdickungsmitteln, auch in Kombination mit weiteren, in der Technologie von pharmazeutischen Suspensionen gängigen Hilfsmitteln und Zusatzstoffen, kaum eine befriedigende Lösung des Problems erkennen ließ.

Um so überraschender war das Ergebnis, das eine Kombination des Arzneistoffes Sucralfat, des Verdickungsmittels Xanthangummi und mindestens eines Peptisators zu einer stabilen, den Anforderungen der Galenik sowie der medizinischen Anwendung in hervorragender Weise gerecht werdenden Sucralfat-Suspension führt, wenn die mengenmäßige Zusammensetzung erfindungsgemäß gewählt wird.

Das für die Herstellung der erfindungsgemäßen Sucralfat-Suspensionen verwendete Sucralfat ist ein gängiger pharmazeutischer Wirkstoff und kommt hier vorzugsweise in fein gemahlener Form mit einer Teilchengröße von unter 50 µm zum Einsatz.

Der erfindungsgemäß als Suspendierungs- und Verdickungsmittel verwendete Xanthangummi kann durch Fermentationsprozesse im technischen Maßstab hergestellt werden und ist den Qualitätsvorschriften der Pharmakopöen der verschiedenen Länder entsprechend im Handel.

Die erfindungsgemäß der Suspension zuzusetzenden Peptisatoren sind Salze anorganischer oder organischer Säuren, die gewährleisten sollen, daß in dispersen Systemen wie den vorliegenden Suspensionen der hochmolekulare Zusatzstoff Xanthangummi im Solzustand, also in homogener Verteilung, verbleibt und sich nicht durch Gelbildung ausscheidet. Dies können beispielsweise sein physiologisch unbedenkliche Salze der Phosphorsäure, der Citronensäure oder anderer dreibasischer Säuren. Insbesondere eignen sich hierfür Salze der Phosphor- und Citronensäure. Besonders bevorzugt ist Natriumdihydrogenphosphat.

Für eine wirksame, dauerhafte Stabilisierung werden erfindungsgemäß den Sucralfat-Suspensionen, bezogen auf den Gehalt an Sucralfat, 1-5 Gew.% Xanthangummi und 1-12,5 Gew.% mindestens eines Peptisators zugesetzt. Der Gehalt an Sucralfat in diesen Zubereitungen kann in weiten Bereichen variieren.

In der Regel können solche Suspensionen 1-40 Gew.% Sucralfat, bezogen auf die Gesamtmenge, enthalten. Typische Formulierungen von Sucralfat-Suspensionen entsprechend der Erfindung enthalten, bezogen auf die Gesamtmenge, 1-40 Gew.% vorzugsweise 10-25 Gew.% Sucralfat, 0,01-2 Gew.%, vorzugsweise 0,1-1 Gew.% Xanthangummi und 0,01-5 Gew.%, vorzugsweise 0,1-3 Gew.% Peptisator. Neben dem Wasser als flüssigem Medium können die erfindungsgemäßen Suspensionen noch viskositätserhöhende Flüssigkeiten, vorzugsweise Glycerin oder Propandiol, in einem Anteil von 1-50 Gew.%, vorzugsweise 10-20 Gew.%, enthalten. Darüberhinaus sind Zusätze von weiteren, in der Technologie von pharmazeutischen Suspensionen üblichen Hilfsmitteln und Zusatzstoffen möglich. Hierzu zählen vornehmlich Konservierungsstoffe wie etwa Natrium-methyl-4-hydroxybenzoat und Natrium-propyl-4-hydroxybenzoat, die gemeinsam oder einzeln in üblichen Konzentrationen etwa um 0,1 Gew.%, zugesetzt werden. Weiterhin können geschmacksverbessernde Aromen, Süßungsmittel und Geschmackskorrigentien zugefügt werden. Derartige Zusätze übersteigen in der Regel kaum einen Gehalt von 1 Gew.%, bezogen auf die Gesamtmenge. Den erfindungsgemäßen Sucralfat-Suspensionen können auch weitere Wirkstoffe zugesetzt werden, beispielsweise solche, mit denen Sucralfat bekanntermaßen kombiniert werden kann, wie etwa Antacida, Spasmolytika, Antiflatulentia $H_2$-Rezeptorenblocker, nichtsteroidale Antirheumatika und allgemein säuresekretionshemmende Pharmaka. Hierzu zählen auch jene in der EP-A1-0107209 beschriebenen Aminosäuren, die die schleimhautauskleidende Wirkung von Sucralfat verstärken bzw. diese bei ungünstigen Lagerbedingungen entsprechender Präparate erhalten sollen.

Die Herstellung der erfindungsgemäßen Sucralfat-Suspensionen erfolgt in an sich bekannter Weise durch Mischen der Komponenten und Homogenisieren. Diese können dann in für pharmazeutische Suspensionen übliche Verpackungsmittel wie etwa Flaschen, Trink-Ampullen oder Portionspackungen zur oralen oder rektalen Applikation abgefüllt werden. Der Wirkstoff bleibt über beliebig lange Lagerzeit hinweg suspendiert, ohne irreversibel zu sedimentieren, ohne zu verklumpen oder sich an den Gefäßwandungen niederzuschlagen.

Der medizinische Anwendungsbereich der erfindungsgemäßen Sucralfat-Suspensionen ist völlig analog zu dem der bekannten Darreichungsformen enthaltend Sucralfat als Wirkstoff, nämlich Schutz und Heilung angegriffener Schleimhäute des Verdauungstraktes beim Menschen, insbesondere Linde-

3

rung der Beschwerden und Heilung bei Ulcus ventriculi et duodeni. Aber auch in der Veterinärmedizin kann das Mittel vorteilhaft bei der Bekämpfung von Emesis und/oder Diarrhoe eingesetzt werden. Üblicherweise wird für entsprechende Behandlungen die Suspension mit einer zu den bekannten Darreichungsformen von Sucralfat analogen Dosierung oral verabfolgt. Falls angezeigt, können derartige Suspensionen aber auch rektal appliziert werden. Dies ist ein besonderer Vorteil der erfindungsgemäßen Sucralfat-Suspensionen, da eine derartige Anwendung mit den bislang bekannten Darreichungsformen praktisch nicht möglich war.

Beispiel 1

5 ml einer Oralsuspension mit 20 Gew.% Sucralfat enthalten

| | |
|---|---|
| Sucralfat | 1,118 g |
| $NaH_2PO_4$ | 0,03 g |
| Xanthangummi | 0,02 g |
| Glycerin | 0,50 g |
| Natrium-methyl-4-hydroxybenzoat | 0,0025 g |
| Natrium-propyl-4-hydroxybenzoat | 0,0025 g |
| Aromastoffe | q.s. |
| Wasser | ad 5,0 ml. |

Beispiel 2

100 ml einer Rektalsuspension mit 10 Gew.% Sucralfat enthalten

| | |
|---|---|
| Sucralfat | 11,18 g |
| $NaH_2PO_4$ | 0,40 g |
| Xanthangummi | 0,19 g |
| Glycerin | 10,00 g |
| Natrium-methyl-4-hydroxybenzoat | 0,05 g |
| Natrium-propyl-4-hydroxybenzoat | 0,05 g |
| Wasser | ad 100 ml |

## Patentansprüche

1. Pharmazeutische Zubereitungen in Form von Sucralfat enthaltenden Suspensionen, gekennzeichnet durch einen Gehalt, bezogen auf das Sucralfat, von 1-5 Gew.% Xanthangummi und 1-12,5 Gew.% mindestens eines Peptisators.

2. Pharmazeutische Zubereitungen nach Anspruch 1, dadurch gekennzeichnet, daß der Peptisator mindestens ein Salz der Phosphor- und/oder Citronensäure ist.

3. Pharmazeutische Zubereitungen nach den Ansprüchen 1 oder 2, dadurch gekennzeichnet, daß sie bezogen auf die Gesamtmenge, 1-40 Gew.% Sucralfat enthalten.

4. Verfahren zur Stabilisierung von pharmazeutischen Zubereitungen in Form von Sucralfat enthaltenden Suspensionen, dadurch gekennzeichnet, daß man diesen Zubereitungen, bezogen auf den Gehalt an Sucralfat, 1-5 Gew.% Xanthangummi und 1-12,5 Gew.% mindestens eines Peptisators zusetzt.

## Claims

1. Pharmaceutical formulations in the form of suspensions containing sucralfate, characterised in that they contain, based on the sucralfate, 1-5% by weight of xanthan gum and 1-12.5% by weight of at least one peptiser.

2. Pharmaceutical formulations according to Claim 1, characterised in that the peptiser is at least one salt of phosphoric acid and/or citric acid.

3. Pharmaceutical formulations according to Claim 1 or 2, characterised in that they contain 1-40% by weight of sucralfate, based on the total amount.

4. Method of stabilising pharmaceutical formulations in the form of suspensions containing sucralfate, characterised in that 1-5% by weight of xanthan gum and 1-12.5% by weight of at least one peptiser, based on the content of sucralfate, are added to these formulations.

## Revendications

1. Compositions pharmaceutiques sous forme de suspensions contenant du Sucralfate, caractéri-

4

sées en ce qu'elles contiennent, par rapport au Sucralfate, de 1 à 5% en poids de gomme de xanthane et de 1 à 12,5% en poids d'au moins un agent peptisant.

2. Compositions pharmaceutiques selon la revendication 1, caractérisées en ce que l'agent peptisant consiste en au moins un sel de l'acide phosphorique et/ou de l'acide citrique.

3. Compositions pharmaceutiques selon les revendications 1 ou 2, caractérisées en ce qu'elles contiennent, par rapport au poids total, de 1 à 40% en poids de Sucralfate.

4. Procédé pour stabiliser des compositions pharmaceutiques sous forme de suspensions contenant du Sucralfate, caractérisé en ce que l'on ajoute à ces compositions, par rapport à la teneur en Sucralfate, de 1 à 5% en poids de gomme de xanthane et de 1 à 12,5% en poids d'au moins un agent peptisant.